(19) Europäisches Patentamt
European Patent Office
Office européen des brevets

(11) **EP 2 518 038 A1**

(12) **EUROPEAN PATENT APPLICATION**
published in accordance with Art. 153(4) EPC


(43) Date of publication:
**31.10.2012 Bulletin 2012/44**

(21) Application number: **10838929.7**

(22) Date of filing: **20.12.2010**

(51) Int Cl.:
***C07C 13/66*** *(2006.01)* ***C07C 255/50*** *(2006.01)*
***C07C 255/51*** *(2006.01)* ***C07F 7/08*** *(2006.01)*
***C09K 11/06*** *(2006.01)* ***H01L 51/50*** *(2006.01)*

(86) International application number:
**PCT/JP2010/007361**

(87) International publication number:
**WO 2011/077689 (30.06.2011 Gazette 2011/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.12.2009 JP 2009289733**



(71) Applicant: **Idemitsu Kosan Co., Ltd.**
**Chiyoda-ku**
**Tokyo 100-8321 (JP)**

(72) Inventors:
- **MIZUKI, Yumiko**
  **Sodegaura-shi**
  **Chiba 299-0293 (JP)**
- **KAWAMURA, Masahiro**
  **Sodegaura-shi**
  **Chiba 299-0293 (JP)**
- **KAWAMURA, Yuichiro**
  **Sodegaura-shi**
  **Chiba 299-0293 (JP)**
- **SAITO, Hiroyuki**
  **Sodegaura-shi**
  **Chiba 299-0293 (JP)**

(74) Representative: **Gille Hrabal**
**Brucknerstrasse 20**
**40593 Düsseldorf (DE)**


(54) **PYRENE DERIVATIVE AND ORGANIC ELECTROLUMINESCENT ELEMENT USING THE SAME**


(57) A pyrene derivative represented by the following formula (1), wherein $Ar_1$, $Ar_2$ and $Ar_3$ are independently a substituted or unsubstituted aryl group, with specific derivatives being excluded.


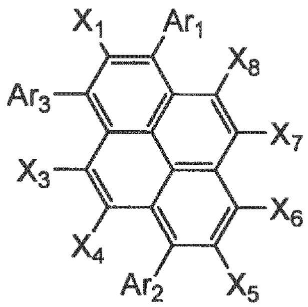

(1)

EP 2 518 038 A1

**Description**

TECHNICAL FIELD

The invention relates to a pyrene derivative and an organic electroluminescence device using the same. More particularly, the invention relates to a pyrene derivative capable of producing an organic electroluminescence device having a high luminous efficiency and a long life.

BACKGROUND ART

An organic electroluminescence (EL) device is a self-emiting device utilizing a principle that a fluorescence material emits light by energy of recombination of holes injected from an anode and electrons injected from a cathode when an electrical field is applied. Such an organic EL device is provided with a pair of electrodes of an anode and a cathode, and an organic luminescence medium between these electrodes.

An organic luminescence medium is formed of a multilayer stack of layers having their respective functions. For example, an organic luminescence medium is a multilayer stack in which an anode, a hole-injecting layer, a hole-transporting layer, an emitting layer, an electron-transporting layer and an electron-injecting layer are sequentially stacked.

As the emitting material of the emitting layer, a material which emits each color (red, green and blue, for example) has been developed. For example, a pyrene derivative is disclosed in Patent Documents 1 to 3 as the blue-emitting material.

However, there is a problem that the pyrene derivatives disclosed in Patent Documents 1 to 3 are not sufficient in respect of luminous efficiency and lifetime.

Related Art Documents

Patent Documents

Patent Document 1: JP-A-2003-234190
Patent Document 2: JP-A-2005-126431
Patent Document 3: JP-A-2009-4351

SUMMARY OF THE INVENTION

The invention is aimed at providing a pyrene derivative capable of producing an organic electroluminescence device having a high luminous efficiency and a long life.

According to the invention, the following pyrene derivative or the like are provided.

1. A pyrene derivative represented by the following formula (1):

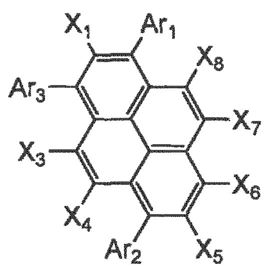

(1)

wherein $Ar_1$, $Ar_2$ and $Ar_3$ are independently a substituted or unsubstituted aryl group; and
$X_1$ and $X_3$ to $X_8$ are independently a hydrogen atom, a substituted or unsubstituted aryl group, a substituted or unsubstituted alkyl group or a substituted or unsubstituted cycloalkyl group;
excluding a derivative in which $X_6$ is a substituted or an unsubstituted aryl group and all of $X_1$ and $X_3$ to $X_5$, $X_7$ and $X_8$ are hydrogen atoms and a derivative in which the substituted or unsubstituted aryl groups of $Ar_1$, $Ar_2$ and $Ar_3$ are the same.

2. The pyrene derivative according to 1, wherein $Ar_1$ and $Ar_2$ are the same and $Ar_3$ is different from $Ar_1$ and $Ar_2$.
3. The pyrene derivative according to 1, wherein $Ar_1$, $Ar_2$ and $Ar_3$ are different from each other.
4. The pyrene derivative according to 1, wherein $X_6$ is a hydrogen atom.
5. The pyrene derivative according to 4, wherein $Ar_1$ and $Ar_2$ are the same.
6. The pyrene derivative according to any of 1 to 5, wherein $Ar_1$ and $Ar_2$ are independently a substituent represented by the following formula (2):

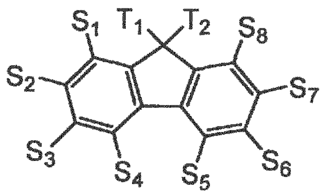
(2)

wherein $S_1$ to $S_8$ are independently a hydrogen atom, a substituted or unsubstituted aryl group, a substituted or unsubstituted alkyl group, a substituted or unsubstituted cycloalkyl group or a substituted or unsubstituted alkenyl group, or at least two adjacent groups of $S_1$ to $S_8$ are combined to form a saturated or unsaturated ring structure that may have a substituent;
$T_1$ and $T_2$ are independently a hydrogen atom, a substituted or unsubstituted aryl group, a substituted or unsubstituted alkyl group, a substituted or unsubstituted cycloalkyl group or a substituted or unsubstituted silyl group; and
the substituent represented by the formula (2) is bonded to the pyrene skeleton of the pyrene derivative through one of $S_1$ to $S_8$ as a single bond or is bonded to the pyrene skeleton of the pyrene derivative by a single bond in any one of bonding positions of a saturated or unsaturated ring structure which is formed by adjacent groups of $S_1$ to $S_8$.
7. The pyrene derivative according to 6, wherein $S_2$ is bonded to the pyrene skeleton of the pyrene derivative represented by the formula (1) as a single bond.
8. The pyrene derivative according to any of 1 to 5, wherein $Ar_1$ and $Ar_2$ are independently a naphthyl group, an aryl group having a naphthyl group, an aryl group having a substituted or unsubstituted silyl group or an aryl group having a cyano group.
9. An organic electroluminescence device comprising a pair of electrodes and one or more organic compound layers comprising an emitting layer therebetween,
wherein the emitting layer comprises the pyrene derivative according to any of 1 to 8 in an amount of 0.1 to 50 mass%.
10. The organic electroluminescence device according to 9, wherein the emitting layer comprises the pyrene derivative in an amount of 0.1 to 20 mass%.

According to the invention, it is possible to provide a pyrene derivative capable of producing an organic electroluminescence device having a high luminous efficiency and a long life.
Further, according to the invention, it is possible to provide an organic EL device having a high luminous efficiency and a long life.

MODE FOR CARRYING OUT THE INVENTION

The pyrene derivative of the invention is represented by the following formula (1): A pyrene derivative represented by the following formula (1):

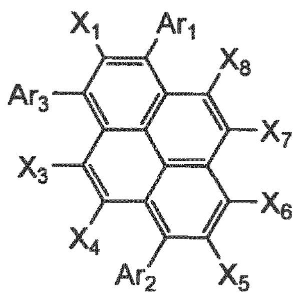
(1)

wherein $Ar_1$, $Ar_2$ and $Ar_3$ are independently a substituted or unsubstituted aryl group; and

$X_1$ and $X_3$ to $X_8$ are independently a hydrogen atom, a substituted or unsubstituted aryl group, a substituted or unsubstituted alkyl group or a substituted or unsubstituted cycloalkyl group;
excluding a derivative in which $X_6$ is a substituted or an unsubstituted aryl group and all of $X_1$ and $X_3$ to $X_5$, $X_7$ and $X_8$ are hydrogen atoms and a derivative in which the substituted or unsubstituted aryl groups of $Ar_1$, $Ar_2$ and $Ar_3$ are the same.

When the pyrene derivative of the invention is used as the dopant of the emitting layer of an organic EL device, for example, a higher luminous efficiency as compared with that attained by conventional dopants can be obtained.

As for the pyrene derivative of the invention, it is preferred that $Ar_1$ and $Ar_2$ be the same and $Ar_3$ differ from $Ar_1$ and $Ar_2$, or that $Ar_1$, $Ar_2$ and $Ar_3$ are different from each other. Here, the "$Ar_1$, $Ar_2$ and $Ar_3$ are different from each other" also means a derivative in which $Ar_1$ to $Ar_3$ including their substituents are different from each other.

In the pyrene derivative of the invention, $X_6$ is a hydrogen atom. More preferably, $X_6$ is a hydrogen atom and $Ar_1$ and $Ar_2$ are the same.

It is preferred that $Ar_1$ and $Ar_2$ of the pyrene derivative of the invention be independently a substituent represented by the following formula (2):

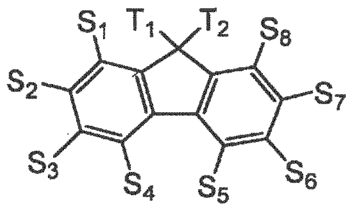
(2)

wherein $S_1$ to $S_8$ are independently a hydrogen atom, a substituted or unsubstituted aryl group, a substituted or unsubstituted alkyl group, a substituted or unsubstituted cycloalkyl group or a substituted or unsubstituted group, or at least two adjacent groups of $S_1$ to $S_8$ are combined to form a saturated or unsaturated ring structure that may have a substituent. $S_1$ to $S_8$ are preferably independently a halogen atom, a substituted or unsubstituted aryl group or a substituted or unsubstituted alkyl group, or at least two adjacent groups of $S_1$ to $S_8$ are combined to form a saturated or unsaturated ring structure. It is more preferred that $S_1$ to $S_8$ be a halogen atom or at least two adjacent groups of $S_1$ to $S_8$ are combined to form a saturated or unsaturated ring structure.

$T_1$ and $T_2$ are independently a hydrogen atom, a substituted or unsubstituted aryl group, a substituted or unsubstituted alkyl group, a substituted or unsubstituted cycloalkyl group and a substituted or unsubstituted silyl group. It is preferred that $T_1$ and $T_2$ be independently a substituted or unsubstituted alkyl group.

The substituent represented by the formula (2) bonds to the pyrene skeleton of the pyrene derivative via any one of $S_1$ to $S_8$ as a single bond, or, in any one of bonding positions of a saturated or unsaturated ring structure formed by bonding of adjacent $S_1$ to $S_8$ by a single bond.

As the structure which is formed when the "at least two adjacent groups of $S_1$ to $S_8$ are bonded to form a saturated or unsaturated ring structure", the following structures can be given, for example.

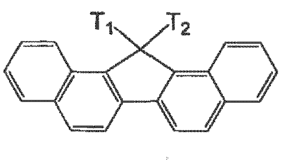
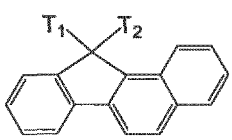
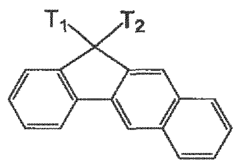

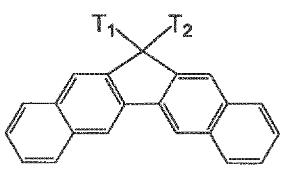
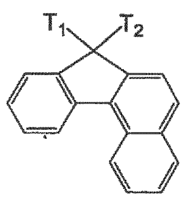
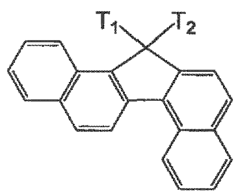

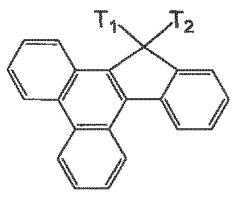
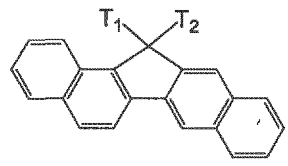
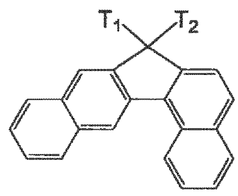

In the substituent represented by the formula (2), it is preferred that $S_2$ be bonded to the pyrene skeleton of the pyrene derivative represented by the formula (1) as a single bond.

Further, it is preferred that $Ar_1$ and $Ar_2$ of the pyrene derivative of the invention be independently ß-naphthyl group (2-naphthyl group), an aryl group having a naphthyl group, an aryl group having a substituted or unsubstituted silyl group, or an aryl group having a cyano group.

Hereinbelow, each substituent of the pyrene derivative of the invention will be explained.

The aryl group represented by $Ar_1$, $Ar_2$, $Ar_3$ and $X_1$, $X_3$ to $X_8$ is preferably an aryl group having 6 to 50 carbon atoms that form a ring (hereinafter referred to as "ring carbon atoms"), more preferably an aryl group having 6 to 20 ring carbon atoms. Examples thereof include a phenyl group, a naphthyl groups, a phenanthryl group, a benzophenanthryl group, an anthryl group, a benzanthryl group, a pyrenyl group, a chrycenyl group, a fluorenyl group, a benzofluorenyl group, a dibenzofluorenyl group, a 9,9-dimethylfluorenyl group, a 9,9-diethylfuorenyl group, a 9,9-dipropylfluorenyl group, 9,9-diisopropylfluorenyl group, a 9,9-dibutylfluorenyl group, a 9,9-diphenylfluorenyl group, a biphenyl group and a triphenylenyl The aryl group is preferably a phenyl group, a naphthyl groups, a phenanthryl group, a biphenyl group, a fluorenyl group, a 9,9-dimethylfluorenyl group, a 9,9-diethylfluorenyl group, a 9,9-dipropylfluorenyl group, a 9,9-diisopropylfluorenyl group, a 9,9-dibutylfluorenyl and a 9,9-diphenylfluorenyl group.

Examples of the alkyl group represented by $X_1$, $X_3$ to $X_8$ include a methyl group, an ethyl group, a propyl group, an isopropyl group, an n-butyl group, an s-butyl group, an isobutyl group, a t-butyl group, an n-pentyl group, an n-hexyl group, an n-heptyl group and an n-octyl group. The alkyl group is preferable a methyl group, an ethyl group, a propyl group, an isopropyl group, an n-butyl group, an s-butyl group, an isobutyl group or a t-butyl group, with a methyl group, an ethyl group, a propyl group, an isopropyl group, and a t-butyl group being particularly preferable. The alkyl group is preferably an alkyl group having 1 to 20 carbon atoms, more preferably an alkyl group having 1 to 10 carbon atoms, with an alkyl group having 1 to 6 carbon atoms being particularly preferable.

Examples of the cycloalkyl group represented by $X_1$, $X_3$ to $X_8$ include a cyclopropyl group, a cyclobutyl group, a cyclopentyl group, a cyclohexyl group, a 4-methylcyclohexyl group, a 1-adamantyl group, a 2-adamantyl group, a 1-norbonyl group and a 2-norbonyl group. Of these, a cyclopentyl group and a cyclohexyl group are preferable. The above-mentioned cycloalkyl group is preferably cycloalkyl group having 3 to 15 carbon atoms, more preferably a cycloalkyl group having 3 to 10 carbon atoms, with a cycloalkyl group having 3 to 6 carbon atoms being particularly preferable.

It is thought that the pyrene derivative of the invention enables an organic EL device to have a long life since it has a substituted or unsubstituted aryl group at a position where the electron density of the pyrene as the mother skeleton is high. It is also thought that the pyrene derivative of the invention enables an organic EL device to have a high luminous efficiency since intermolecular association is suppressed due to the presence of the aryl group.

In the invention, the "aryl group" means a "group which is obtained by removing a hydrogen atom from an aromatic compound", and includes not only a monovalent aryl group but also an "arylene group" which is a divalent group. The hydrogen atom of the compound of the invention includes light hydrogen and heavy hydrogen.

If each of the substituents of $Ar_1$, $Ar_2$, $Ar_3$ and $X_1$ and $X_3$ to $X_8$ has a further substituent, as such a substituent, a substituted or unsubstituted alkyl group, a substituted or unsubstituted cycloalkyl group, a substituted or unsubstituted aryl group, a substituted or unsubstituted alkenyl group, a substituted or unsubstituted silyl group, a cyano group, a halogen atom or the like can be mentioned, for example. Preferably, the substituent is a substituted or unsubstituted aryl group, a substituted or unsubstituted silyl group or a cyano group.

The "further substituent of each of the substituents" means a substituent of the aryl group when the "substituted or unsubstituted aryl group" is a substituted aryl for example. Specifically, $S_1$ to $S_8$, $T_1$ and $T_2$ in the substituent represented by the formula (2) correspond to such a further substituent.

The specific examples of the substituted or unsubstituted alkyl group, the substituted or unsubstituted cycloalkyl group and the substituted or unsubstituted aryl group are as mentioned above.

As the above-mentioned alkenyl group, a vinyl group, an allyl group, a 1-butenyl group, a 2-butenyl group, a 3-butenyl group, a 1,3-butanedienyl group, a 1-methylvinyl group, a styryl group, a 2,2-diphenylvinyl group, 1,2-diphenylvinyl group, a 1-methylallyl group, a 1,1-dimethylallyl group, a 2-methylallyl group, a 1-phenylallyl group, a 2-phenylallyl group, a 3-phenylallyl group, a 3,3-diphenylallyl group, a 1,2-dimethylallyl group, a 1-phenyl-1-butenyl group, a 3-phenyl-1-butenyl group or the like can be mentioned. Preferred examples include a styryl group, a 2,2-diphenylvinyl group, and a 1,2-diphenylvinyl group. The alkenyl group is preferably an alkenyl group having 2 to 30 carbon atoms, more preferably

an alkenyl group having 2 to 20 carbon atoms, with an alkenyl group having 2 to 10 carbon atoms being particularly preferable.

As the above-mentioned substituted group, a trimethylsilyl group, a triethylsilyl group, a t-butyldimethylsilyl group, a vinyldimethylsilyl group, an isopropyldimethylsilyl group, a triphenylsilyl group, a triisopropylsilyl group or the like can be mentioned. The substituted silyl group is preferably a trimethylsilyl group, triethylsilyl group and a t-butyldimethylsilyl group.

As the above-mentioned halogen atom, fluoride, chlorine, bromine, iodine or the like can be mentioned, with a fluorine atom being preferable.

Specific examples of the further substituent of each of the substituents represented by $Ar_1$, $Ar_2$, $Ar_3$ and $X_1$, $X_3$ to $X_8$ include a methyl-substituted phenyl group, a trimethylsilyl-substituted phenyl group, a cyano-substituted phenyl groups, a fluorine-substituted phenyl group, a naphthyl-substituted phenyl group, a cyclohexyl-substituted phenyl group, a trimethylsilyl-substituted fluorenyl group and a phenyl-substituted dibenzo-9,9-dimethylfluorenyl group.

Specific examples of the pyrene derivative of the invention will be explained.

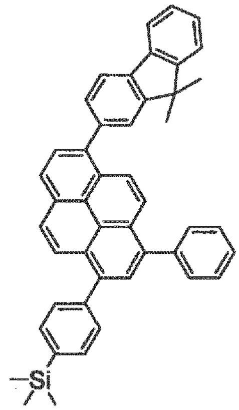
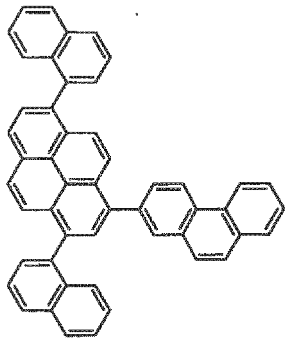
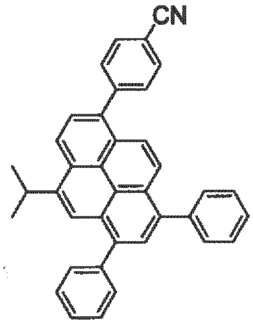
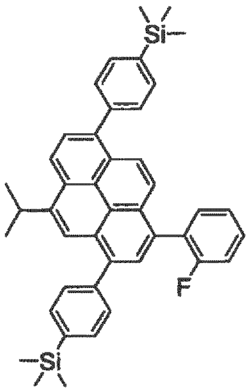
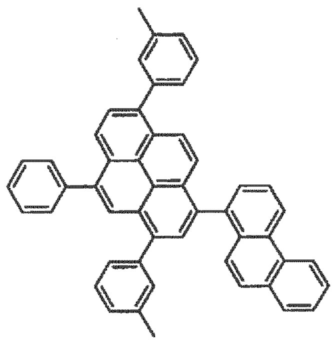
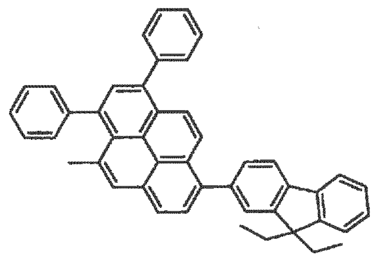
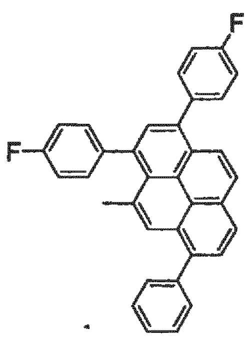
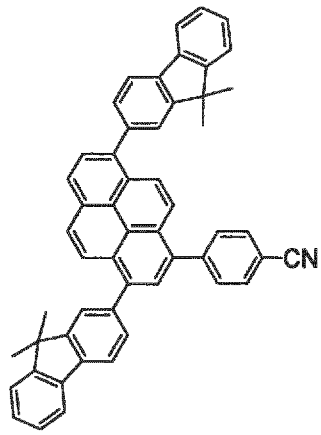
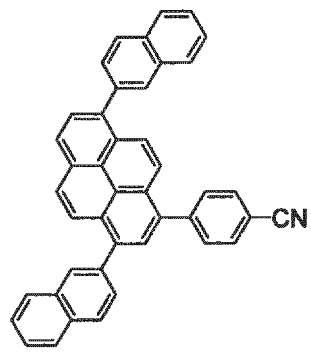

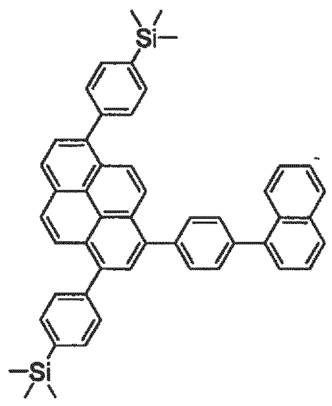
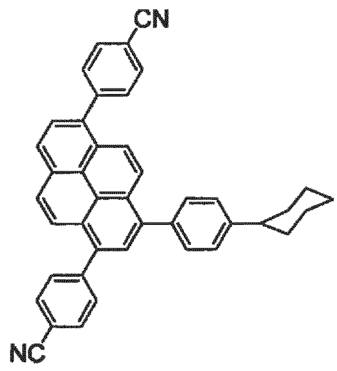
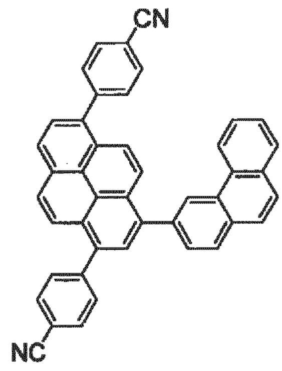

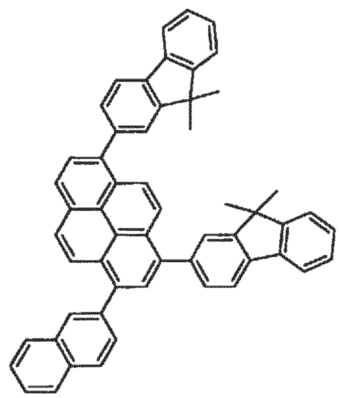
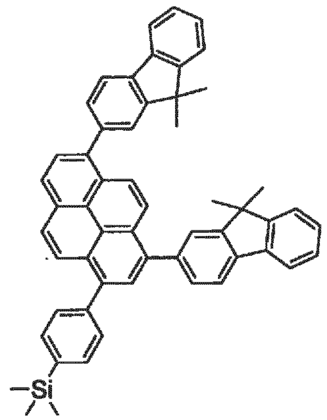
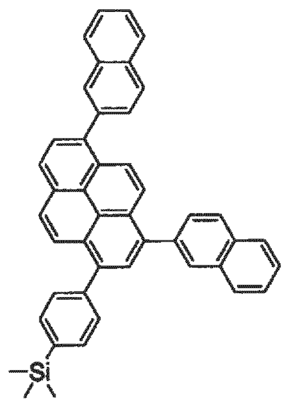

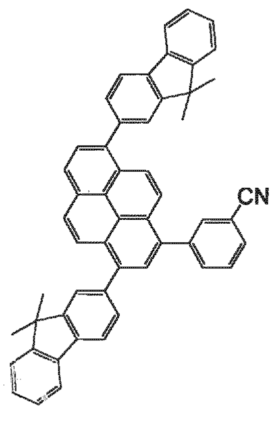
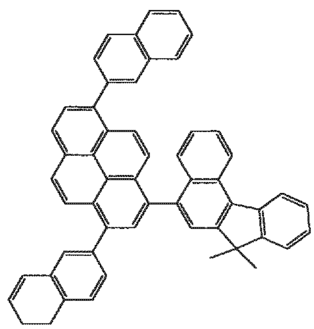
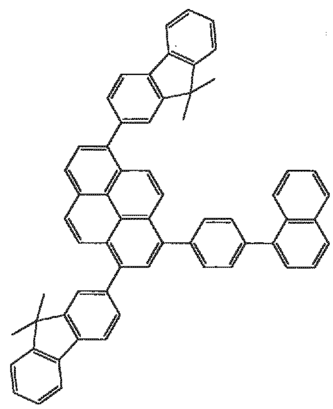

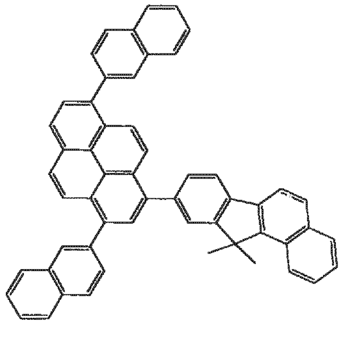
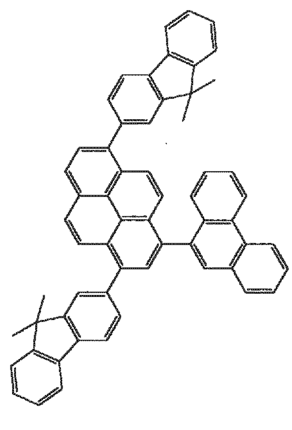
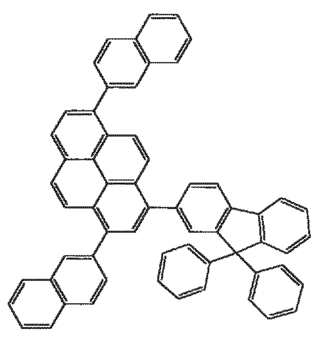

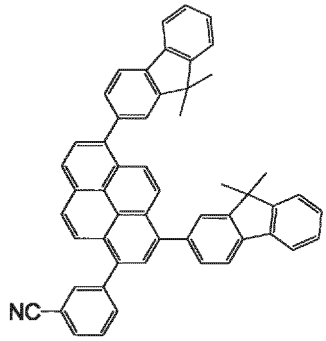

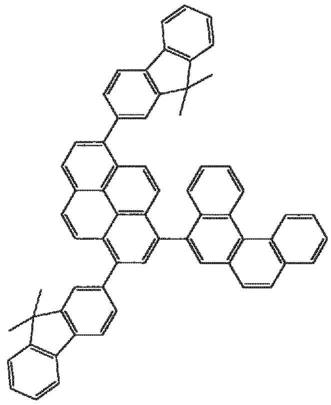

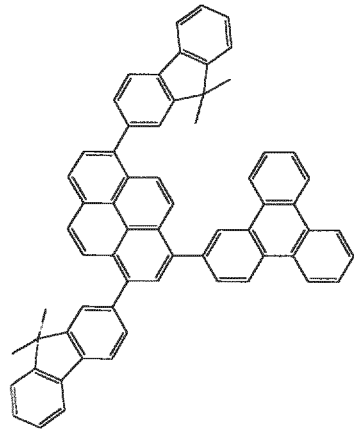

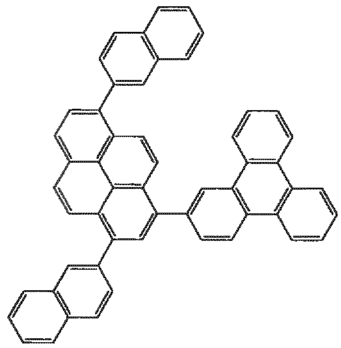

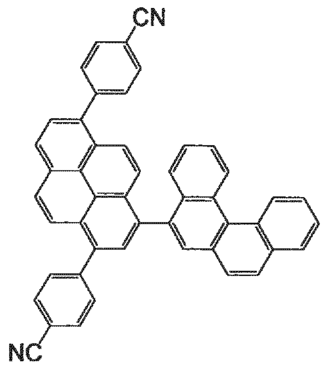

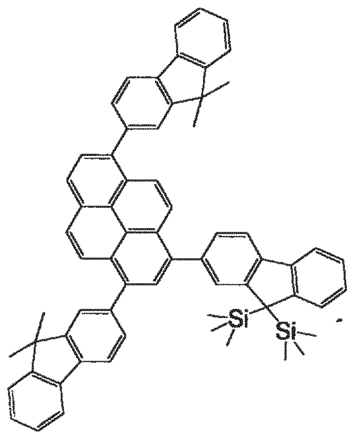

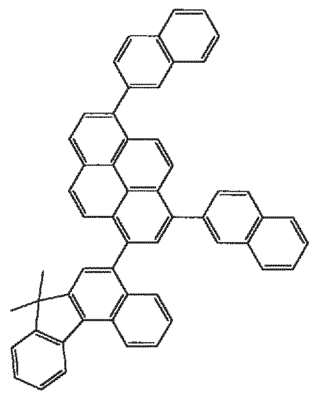

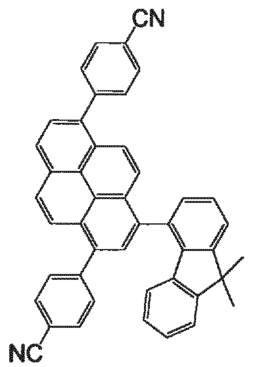

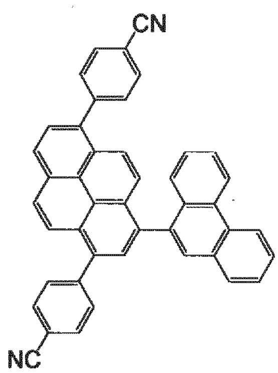

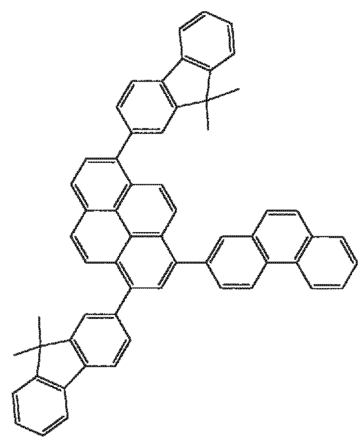

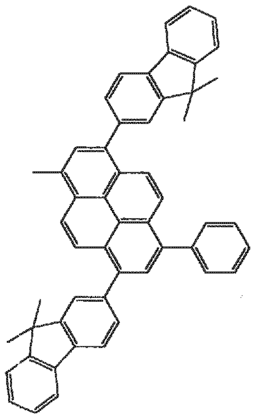

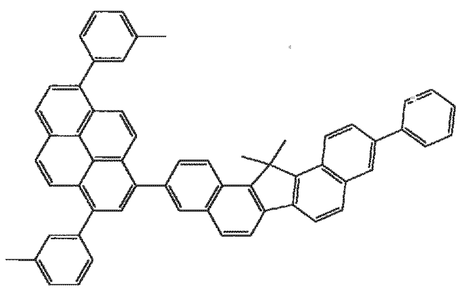

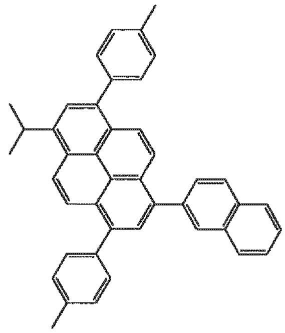

As for the pyrene derivative of the invention, a precursor thereof can be obtained by a Suzuki coupling reaction or the like by using as starting materials a halogenated pyrene compound and an arylboronic acid compound or a halogenated aryl compound and a pyrenylboronic acid compound, which are synthesized by a known method. The pyrene derivative of the invention can be obtained by subjecting the thus obtained precursor to a halogenation reaction, a boronation reaction and a Suzuki coupling reaction in an appropriately combined manner.

Many reports have been made on the above-mentioned Suzuki coupling reaction (Chem. Rev., Vol. 95, No. 7, 2457 (1995)). The reaction can be conducted under the reported conditions.

No specific restrictions are made on the halogenation agent used in the above-mentioned halogenation reaction. However, N-halogenated succinimide is preferably used. The amount of the halogenation agent is normally 0.8 to 10 molar equivalents, preferably 1 to 5 molar equivalents, relative to the base.

The boronation reaction can be conducted by a known method (pages 61 to 90 of vol. 24 of the Fourth Series of Experimental Chemistry edited by the Chemical Society of Japan or J. Org. Chem., Vol. 60, 7508 (1995) or the like).

The pyrene derivative of the invention is preferably used as a material for an organic EL device. It is further preferred that the pyrene derivative of the invention be used as an emitting material, ion particular, a doping material, of an organic EL device.

Regarding the organic EL device of the invention, in the organic EL device in which one or a plurality of organic compound layers including an emitting layer are held between a pair of electrodes, the emitting layer comprises the pyrene derivative of the invention.

In the organic EL device of the invention, the pyrene derivative of the invention is contained preferably in an amount of 0.1 to 50 mass%, further preferably 0.1 to 20 mass%, particularly preferably 0.1 to 18 mass%, particularly preferably 1 to 10 mass%, and most preferably 2.5 to 15 mass%.

The organic EL device using a material for an organic EL device containing the pyrene derivative of the invention can emit blue light.

If the pyrene derivative of the invention is used as the emitting material of an organic EL device, it is preferred that the emitting layer contain at least one of the pyrene derivative represented by the formula (1) and at least one selected from the anthracene derivative represented by the following formula (3) or the pyrene derivative represented by the formula (4). It is preferred that the derivative represented by the following formula (3) or (4) be a host material.

The anthracene derivative represented by the formula (3) is the following compound.

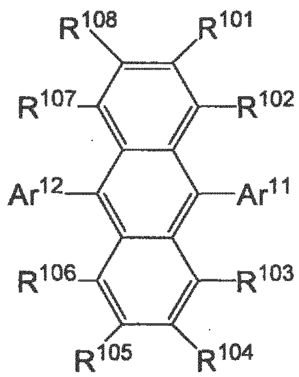

(3)

In the formula (3), $Ar^{11}$ and $Ar^{12}$ are independently a substituted or unsubstituted monocyclic group having 5 to 50 atoms that form a ring (hereinafter referred to as "ring atoms"), a substituted or unsubstituted fused cyclic group having 8 to 50 ring atoms or a group formed of a combination of a monocyclic group and a fused cyclic group; $R^{101}$ to $R^{108}$ are independently an atom or a group selected from a hydrogen atom, a substituted or unsubstituted monocyclic group having 5 to 50 ring atoms, a substituted or unsubstituted fused cyclic group having 8 to 50 ring atoms, a group formed of a monocyclic group and a fused cyclic group, a substituted or unsubstituted alkyl group having 1 to 50 carbon atoms, a substituted or unsubstituted cycloalkyl group having 3 to 50 ring carbon atom, a substituted or unsubstituted alkoxy group having 1 to 50 carbon atoms, a substituted or unsubstituted aralkyl group having 7 to 50 carbon atoms, a substituted or unsubstituted aryloxy group having 6 to 50 ring carbon atoms, a substituted or unsubstituted silyl group, a halogen atom and a cyano group.

In the formula (3), the monocylic group is a group formed only of a ring structure having no fused structure. Specific preferable examples of the monocyclic group having 5 to 50 ring atoms (preferably 5 to 30 ring atoms, more preferably 5 to 20 ring atoms) include aromatic groups such as a phenyl groups, a biphenyl group, a terphenyl group and a quarterphenyl group and heterocyclic groups a such as a pyridyl group, a pyrazyl group, a pyrimidyl group, a triazinyl group, a furyl group and a thienyl group.
Of these, a phenyl group, a biphenyl group and a terphenyl group are preferable.

In the formula (3), the fused cyclic group is a group formed by fusing two or more ring structures.
Specifically, as examples of the fused cyclic group having 8 to 50 ring atoms (preferably, 8 to 30 ring atoms, more preferably 8 to 20 ring atoms), a fused aromatic group such as a naphthyl group, a phenanthryl group, an anthryl group, a chrycenyl group, a benzanthryl group, a benzophenanthryl group, a triphenylenyl group, a benzochryceny group, an indenyl group, a fluorenyl group, 9,9-dimethylfluorenyl group, a benzofluorenyl group, a dibenzofluorenyl group, a fluoranthenyl group or a benzofluoranthenyl group or a fused heterocyclic group such as a benzofuranyl group, a benzothiophenyl group, an indolyl group, a dibenzofuranyl group, a dibenzothiophenyl group, a carbazolyl group, a quinolyl group and a phenanthrolinyl group are preferable.
Of these, a naphthyl group, a phenanthryl group, an anthryl group, a 9,9-dimethylfluorenyl group, a fluoranthenyl group, a benzanthryl group, a dibenzothiophenyl group, a dibenzofuranyl group and a carbazolyl group are preferable.

The specific examples of the alkyl group, the substituted silyl group, the cyclcoalkyl group and the halogen atom in the formula (3) are the same as the specific examples of each group represented by $X_1$, $X_3$ to $X_8$ in the formula (1) and the substituent in the "further substituent of each of the substituents".

The alkoxy group is represented by -OY Examples of Y include the examples of the above-mentioned alkyl group. The alkoxy group may be a methoxy group and an ethoxy group.
The aryloxy group is represented by —OZ. Examples of Z include the examples of the above-mentioned aryl group or the examples of the monocyclic group and the fused cyclic group which will be mentioned later. The aryloxy group is a phenoxy group, for example.

The aralkyl group is represented by —Y-Z, and examples of Y include alkylene groups corresponding to the examples of the alkyl group, and examples of Z are the same as those of the aryl group. The aralkyl group is an aralkyl group having 7 to 50 carbon atoms (the aryl part has 6 to 49 (preferably 6 to 30, more preferably 6 to 20, and particularly preferably 6 to 12) carbon atoms, the alkyl part has 1 to 44 (preferably 1 to 30, more preferably 1 to 20, further preferably 1 to 10 and particularly preferably 1 to 6) carbon atoms). The aralkyl group is a benzyl group, a phenylethyl group or a 2-phenylpropane-2-yl group, for example.

As the substituents of the "substituted or unsubstituted" groups represented by $Ar^{11}$, $Ar^{12}$ and $R^{101}$ to $R^{108}$, a monocyclic group, a fused cyclic group, an alkyl group, a cycloalkyl group, a substituted or unsubstituted silyl group, an alkoxy group, a cyano group and a halogen atom (fluorine, in particular) are preferable. A monocyclic group and a fused cyclic are particularly preferable. Specific examples of preferable substituents are the same as the groups in the formula (3) and the groups in the formula (1).

It is preferred that the anthracene derivative represented by the formula (3) be any of the following anthracene derivatives (A), (B) and (C). A preferable anthracene derivative represented by the formula (3) is selected according to the constitution or required properties of an organic EL device to which the anthracene derivative is applied.

(Anthracene derivative (A))

In this anthracene derivative, $Ar^{11}$ and $Ar^{12}$ in the formula (3) are independently a substituted or unsubstituted fused cyclic group having 8 to 50 ring atoms. This anthracene derivative can be divided into a derivative in which $Ar^{11}$ and $Ar^{12}$ are the same substituted or unsubstituted fused cyclic group and a derivative in which $Ar^{11}$ and $Ar^{12}$ are the different substituted or unsubstituted fused cyclic groups.

An anthracene derivative in which $Ar^{11}$ and $Ar^{12}$ in the formula (3) are different (including the difference in substitution position) substituted or unsubstituted fused cyclic group is particularly preferable. Specific preferable examples of the fused cyclic group are as mentioned above. Of these, a naphthyl group, a phenanthryl group, a benzanthryl

group, a 9,9-dimethylfluorenyl group and a dibenzofuranyl group are preferable.

(Anthracene derivative (B))

In this anthracene derivative, one of $Ar^{11}$ and $Ar^{12}$ in the formula (3) is a substituted or unsubstituted fused monocyclic group having 5 to 50 ring atoms and the other is a substituted or unsubstituted fused cyclic group having 8 to 50 ring atoms.
In a preferred mode, $Ar^{12}$ is a naphthyl group, a phenanthryl group, a benzanthryl group, a 9,9-dimethylfluorenyl group or a dibenzofuranyl group, and $Ar^{11}$ is a phenyl group which is substituted by a monocyclic group or a fused cyclic group. Specific examples of a preferable monocyclic group and a fused cyclic group are as mentioned above.
In another preferable mode, $Ar^{12}$ is a fused cyclic group and $Ar^{11}$ is an unsubstituted phenyl group. In this case, as the fused cyclic group, a phenanthryl group, a 9,9-dimethylfluorenyl group, a dibenzofuranyl group and a benzoanthryl group are particularly preferable.

(Anthracene derivative (C))

In this anthracene derivative, $Ar^{11}$ and $Ar^{12}$ in the formula (3) are independently a substituted or unsubstituted monocyclic group having 5 to 50 ring atoms.
In a preferred mode, $Ar^{11}$ and $Ar^{12}$ are both a substituted or unsubstituted phenyl group.
In a further preferred mode, the anthracene derivative (C) is divided into a derivative in which $Ar^{11}$ is an unsubstituted phenyl group and $Ar^{12}$ is a phenyl group having a monocyclic group or a fused cyclic group as a substituent and a derivative in which $Ar^{11}$ and $Ar^{12}$ are independently a phenyl group having a monocyclic group or a fused cyclic group. Specific examples of the monocyclic group or the fused cyclic group which is preferable as the substituent are as mentioned above. As the monocyclic group as the substituent, a phenyl group and a biphenyl group are further preferable, and as the fused cyclic group as the substituent, a naphthyl group, a phenanthryl group, a 9,9-dimethylfluronenyl group, a dibenzofuranyl group and a benzanthryl group are preferable.
The pyrene derivative represented by the formula (4) is the following compound.

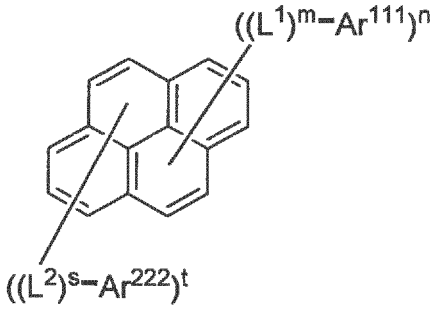

(4)

In the formula (4), $Ar^{111}$ and $Ar^{222}$ are independently a substituted or unsubstituted aryl group having 6 to 30 ring carbon atoms.
$L^1$ and $L^2$ are independently a substituted or unsubstituted divalent aryl group having 6 to 30 ring carbon atoms or a heterocyclic group.
m is an integer of 0 to 1, n is an integer of 1 to 4, s is an integer of 0 to 1 and t is an integer of 0 to 3.
$L^1$ bonds to any of the $1^{st}$ to $5^{th}$ positions of the pyrene, and $L^2$ or $Ar^{222}$ bonds to any of the $6^{th}$ to $10^{th}$ positions of the pyrene.
$L^1$ and $L^2$ in the general formula (4) is preferably a divalent aryl group formed of a substituted or unsubstituted phenylene group, a substituted or unsubstituted biphenylene group, a substituted or unsubstituted naphthylene group, a substituted or unsubstituted terphenylene group, a substituted or unsubstituted fluorenylene group or a combination of these substituents.
As the substituent, the same substituents as those given in the "further substituent of each of the substituents" in the above-mentioned formula (1) can be given. The substituents $L^1$ and $L^2$ are preferably an alkyl group having 1 to 20 carbon atoms.
m in the general formula (4) is preferably an integer of 0 to 1. n in the general formula (4) is preferably an integer of 1 to 2. s in the general formula (4) is preferably an integer of 0 to 1.
t in the general formula (4) is preferably an integer of 0 to 2.
The aryl groups of $Ar^{111}$ and $Ar^{222}$ are the same as those in the above-mentioned formula (1).
The aryl groups of $Ar^{111}$ and $Ar^{222}$ are preferably a substituted or unsubstituted aryl group having 6 to 20 ring carbon

atoms, more preferably a substituted or unsubstituted aryl group having 6 to 16 ring carbon atoms. Specific examples of the aryl group include an phenyl group, a naphthyl group, a phenanthryl group, a fluorenyl group, a biphenyl group, an anthryl group and a pyrenyl group.

In the organic EL device of the invention, each organic layer such as an emitting layer can be formed by a dry film-forming method such as vacuum vapor deposition, molecular beam epitaxy (MBE), sputtering, plasma ion coating, ion plating or the like or a coating method such as spin coating, dipping, casting, bar coating, roll coating, flow coating, ink jetting or the like of a solution obtained by dissolving in a solvent.

In particular, when an organic EL device is produced by using the pyrene derivative of the invention, an organic compound layer and an emitting layer can be formed not only by deposition but also by a wet method.

Although the film thickness of each of the organic compound layers is not particularly limited, it is required to adjust the film thickness to an appropriate value. If the film thickness is too small, pinholes or the like are generated, and a sufficient luminance cannot be obtained even if an electrical field is applied. If the film thickness is too large, a large voltage is required to be applied in order to obtain a certain optical 1 output, which results in a poor efficiency. The suitable film thickness is normally 5 nm to 10 $\mu$m, with a range of 10 nm to 0.2 $\mu$m being further preferable.

The pyrene derivative of the invention and the anthracene derivative (3) or the pyrene derivative (4) mentioned above can be used in the hole-injecting layer, the hole-transporting layer, the electron-injecting layer and the electron-transporting layer in addition to the emitting layer.

In the invention, as the organic EL device in which the organic compound layer (organic thin film layer) is composed of plural layers, one in which layers are sequentially stacked (anode/hole-injecting layer/emifting layer/cathode), (anode/emitting layer/electron-injecting layer/cathode), (anode/hole-injecting layer/emitting layer/electron-injecting layer/cathode), (anode/hole-injecting layer/hole-transporting layer/emitting layer/electron-injecting layer/cathode) or the like can be given.

By allowing the organic thin film layer to be composed of plural layers, the organic EL device can be prevented from lowering of luminance or lifetime due to quenching. If necessary, an emitting material, a doping material, a hole-injecting material or an electron-injecting material can be used in combination. Further, due to the use of a doping material, luminance or luminous efficiency may be improved. The hole-injecting layer, the emitting layer and the electron-injecting layer may respectively be formed of two or more layers. In such a case, in the hole-injecting layer, a layer which injects holes from an electrode is referred to as a hole-injecting layer, and a layer which receives holes from the hole-injecting layer and transports the holes to the emitting layer is referred to as a hole-transporting layer. Similarly, in the electron-injecting layer, a layer which injects electrons from an electrode is referred to as an electron-injecting layer and a layer which receives electrons from an electron-injecting layer and transports the electrons to the emitting layer is referred to as an electron-transporting layer. Each of these layers is selected and used according to each of the factors of a material, i.e. the energy level, heat resistance, adhesiveness to the organic layer or the metal electrode or the like.

Examples of the material other than the derivative represented by the formula (3) or (4) which can be used in the emitting layer together with the pyrene derivative of the invention include, though not limited thereto, fused polycyclic aromatic compounds such as naphthalene, phenanthrene, rubrene, anthracene, tetracene, pyrene, perylene, chrysene, decacyclene, coronene, tetraphenylcyclopentadiene, pentaphenylcyclopentadiene, fluorene and spirofluorene and derivatives thereof, organic metal complexes such as tris(8-quinolinolate)aluminum, triarylamine derivatives, styrylamine derivatives, stilbene derivatives, coumarin derivatives, pyrane derivatives, oxazone derivatives, benzothiazole derivatives, benzoxazole derivatives, benzimidazole derivatives, pyrazine derivatives, cinnamate derivatives, diketo-pyrrolopyrrole derivatives, acrylidone derivatives and derivatives.

As the hole-injecting material, a compound which can transport holes, exhibits hole-injecting effects from the anode and excellent hole-injection effect for the emitting layer or the emitting material, and has an excellent capability of forming a thin film is preferable. Specific examples thereof include, though not limited thereto, phthalocyanine derivatives, naphthalocyanine derivatives, porphyline derivatives, benzidine-type triphenylamine, diamine-type triphenylamine, hexacyanohexaazatriphenylene, derivatives thereof, and polymer materials such as polyvinylcarbazole, polysilane and conductive polymers.

Of the hole-injecting materials usable in the organic EL device of the invention, further effective hole-injecting materials are phthalocyanine derivatives.

Examples of the phthalocyanine (Pc) derivative include, though not limited thereto, phthalocyanine derivatives such as $H_2$Pc, CuPc, CoPc, NiPc, ZnPc, PdPc, FePc, MnPc, ClAlPc, ClGaPc, ClInPc, ClSnPc, $Cl_2$SiPc, (HO)AlPc, (HO) GaPc, VOPc, TiOPc, MoOPc and GaPc-O-GaPc, and naphthalocyanine derivatives.

In addition, it is also possible to sensitize carriers by adding to the hole-injecting material an electron-accepting substance such as a TCNQ derivative.

Preferable hole-transporting materials usable in the organic EL device of the invention are aromatic tertiary amine derivatives.

Examples of the aromatic tertiary in derivative include, though not limited thereto, N,N'-diphenyl-N,N'-dinaphthyl-1,1'-biphenyl-4,4'-diamine, N,N,N',N'-tetrabiphenyl-1,1'-biphenyl-4,4'-diamine or an oligomer or a polymer having these aromatic tertiary amine skeletons.

As the electron-injecting material, a compound which can transport electrons, exhibits electron-injecting effects from the cathode and excellent electron-injection effect for the emitting layer or the emitting material, and has an excellent capability of forming a thin film is preferable.

In the organic EL device of the invention, further effective electron-injecting materials are a metal complex compound and a nitrogen-containing heterocyclic derivative.
Examples of the metal complex compound include, though not limited thereto, 8-hydroxyquinolinate lithium, bis(8-hydroxyquinolinate)zinc,tris(8-hydroxyquinolinate)aluminum, tris(8-hydroxyquinolinate)gallium, bis(10-hydroxybenzo[h]quinolinate)beryllium and bis(10-hydroxybenzo[h]quinolinate)zinc.

As examples of the nitrogen-containing heterocyclic derivative, oxazole, thiazole, oxadiazole, thiadiazole, triazole, pyridine, pyrimidine, triazine, phenanthroline, benzimidazole, imidazopyridine or the like are preferable, for example. Of these, a benzimidazole derivative, a phenanthroline derivative and an imidazopyridine derivative are preferable.
As a preferred mode, a dopant is further contained in these electron-injecting materials. In order to facilitate receiving electrons from the cathode, it is more preferable to dope the vicinity of the cathode interface of the second organic layer with a dopant, the representative example of which is an alkali metal.
As the dopant, a donating metal, a donating metal compound and a donating metal complex can be given. These reducing dopants may be used singly or in combination of two or more.

In the organic EL device of the invention, the emitting layer may contain, in addition to at least one selected from the pyrene derivatives represented by the formulas (1), at least one of an emitting material, doping material, hole-injecting material, hole-transporting material and electron-injecting material in the same layer. Moreover, for improving stability of the organic EL device obtained by the invention to temperature, humidity, atmosphere, etc. it is also possible to prepare a protective layer on the surface of the device, and it is also possible to protect the entire device by applying silicone oil, resin, etc.

As the conductive material used in the anode of the organic EL device of the invention, a conductive material having a work function of more than 4 eV is suitable. Carbon, aluminum, vanadium, iron, cobalt, nickel, tungsten, silver, gold, platinum, palladium or the like, alloys thereof, oxidized metals which are used in an ITO substrate and a NESA substrate such as tin oxide and indium oxide and organic conductive resins such as polythiophene and polypyrrole are used. As the conductive material used in the cathode, a conductive material having a work function of smaller than 4 eV is suitable. Magnesium, calcium, tin, lead, titanium, yttrium, lithium, ruthenium, manganese, aluminum, and lithium fluoride or the like, and alloys thereof are used, but not limited thereto, Representative examples of the alloys include, though not limited thereto, magnesium/silver alloys, magnesium/indium alloys and lithium/aluminum alloys. The amount ratio of the alloy is controlled by the temperature of the deposition source, atmosphere, vacuum degree or the like, and an appropriate ratio is selected. If necessary, the anode and the cathode each may be composed of two or more layers.

In the organic EL device of the invention, in order to allow it to emit light efficiently, it is preferred that at least one of the surfaces be fully transparent in the emission wavelength region of the device. In addition, it is preferred that the substrate also be transparent. The transparent electrode is set such that predetermined transparency can be ensured by a method such as deposition or sputtering by using the above-mentioned conductive materials, It is preferred that the electrode on the emitting surface have a light transmittance of 10% or more. Although no specific restrictions are imposed on the substrate as long as it has mechanical and thermal strength and transparency, a glass substrate and a transparent resin film can be given.

Each layer of the organic EL device of the invention can be formed by a dry film-forming method such as vacuum vapor deposition, sputtering, plasma ion coating, ion plating or the like or a wet film-forming method such as spin coating, dipping, flow coating or the like. Although the film thickness is not particularly limited, it is required to adjust the film thickness to an appropriate value. If the film thickness is too large, a large voltage is required to be applied in order to obtain a certain optical output, which results in a poor efficiency. If the film thickness is too small, pinholes or the like are generated, and a sufficient luminance cannot be obtained even if an electrical field is applied. The suitable film thickness is normally 5 nm to 10 $\mu$m, with a range of 10 nm to 0.2 $\mu$m being further preferable.

In the case of the wet film-forming method, a thin film is formed by dissolving or dispersing materials forming each layer in an appropriate solvent such as ethanol, chloroform, tetrahydrofuran and dioxane. Any of the above-mentioned solvents can be used.
As the solvent suited to such a wet film-forming method, a solution containing the pyrene derivative of the invention as an organic EL material and a solvent can be used.

It is preferred that the organic EL material contain a host material and a dopant material, that the dopant material be the pyrene derivative of the invention, and that the host material be at least one selected from the compounds represented by the formula (3).

In each organic thin film layer, an appropriate resin or additive may be used in order to improve film-forming properties, to prevent generation of pinholes in the film, or for other purposes.

The organic EL device of the invention can be suitably used as a planar emitting body such as a flat panel

display of a wall-hanging television, backlight of a copier, a printer or a liquid crystal display, light sources for instruments, a display panel, navigation light, or the like. The compound of the invention can be used not only in an organic EL device but also in the field of an electrophotographic photoreceptor, a photoelectric converting element, a solar cell and an image sensor.

## EXAMPLES

### Example 1

Compound D-1 was synthesized according to the following scheme:

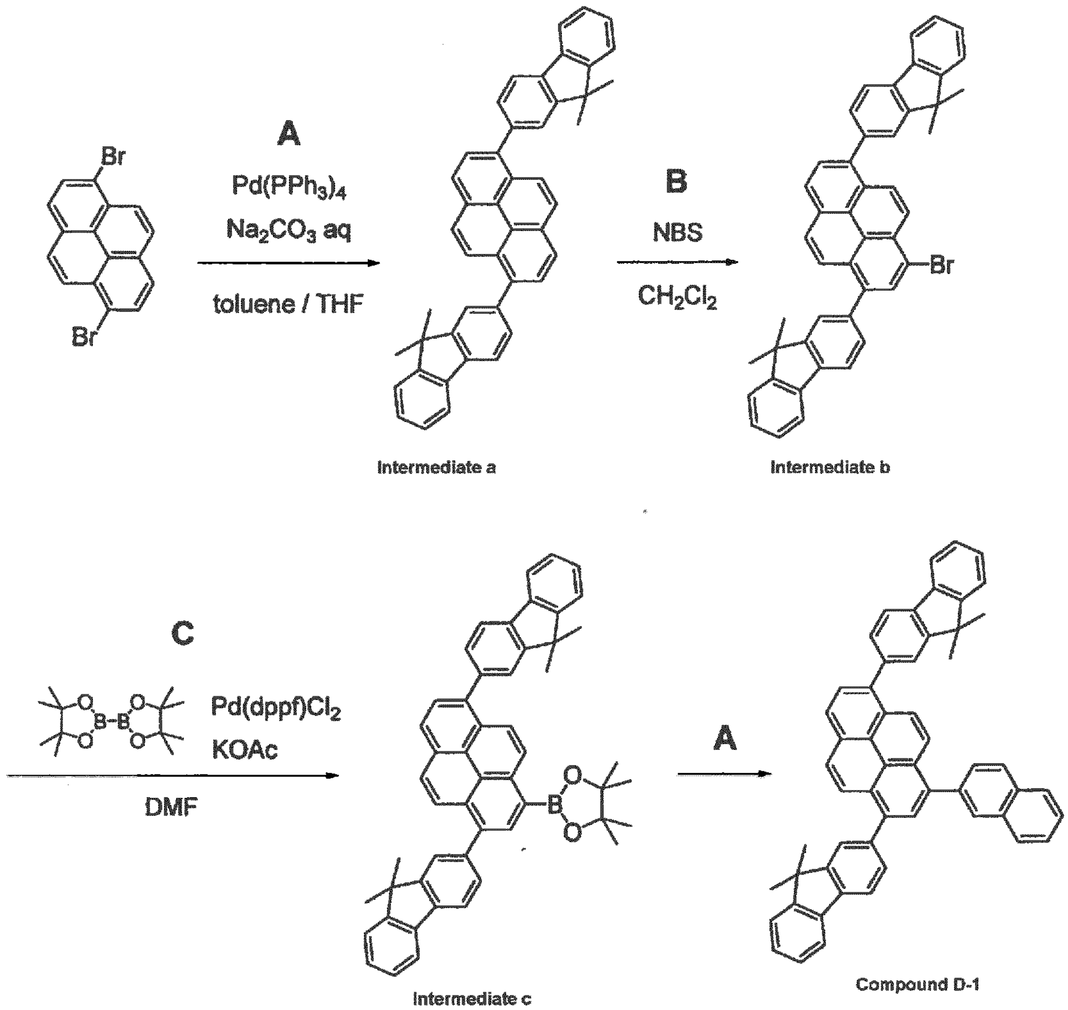


Intermediate a

Intermediate b

Intermediate c

Compound D-1

[Synthesis of intermediate a (synthesis route A)]

Under a flow of argon, in a 1000 mL-recovery flask, 15.0 g (41.6 mmol) of 1,6-dibromopyrene, 25.8 g of 9,9-dimethylfluorene-2-ylboronic acid, 1.9 g (1.67 mmol) of tetrakis(triphenylphosphine)palladium (0) [$Pd(PPh_3)_4$], 27.8 g (262 mmol) of sodium carbonate (130 mL of clean water), toluene and tetrahydrofuran were placed, and the resulting mixture was allowed to react at 90°C for 7 hours. After cooling, the reaction solution was filtered, and solids obtained were washed with methanol and clean water. Further, the solids were purified by silica gel chromatography (heated toluene) and concentrated. . The resulting crude product was re-crystallized from toluene, followed by drying under a reduced pressure, whereby 24.0 g of white solids were obtained.
As a result of a FD-MS (Field desorption mass spectrometry) analysis, the resulting white solids were identified as intermediate a.

[Synthesis of intermediate b (synthesis route B)]

Under a flow of argon, in a 2000 mL-recovery flask, 5.2 g (8.8 mmol) of intermediate a, 1.4 g (7.9 mmol) of N-bromosuccinimide, iodine (a fraction) and dichloromethane were placed. The resulting mixture was allowed to react under reflux for 2 days. After cooling, clean water was added to the reaction solution, and the resulting mixture was separated, followed by extraction. An organic phase was washed with clean water, an aqueous sodium thiosulfate solution and saturated saline, and dried with sodium sulfate, followed by concentration. Crystals obtained by the concentration were washed with methanol, and then dried under a reduced pressure, whereby 5.1 g of white solids were obtained.
As a result of a FD-MS analysis, the resulting white solids were identified as intermediate b.

[Synthesis of intermediate c (synthesis route C)]

Under a flow of argon, in a 1000 mL-recovery flask, 5.1 g (7.6 mmol) of intermediate b, 2.9 g (11.4 mmol) of bis(pinacolato)diboron, 190 mg (0.23 mmol) of [1,1-bis(diphenylphosphino)ferrocene] palladium (II) dichloride, 1.5 g (15.3 mmol) of potassium acetate and dimethylformamide were placed. The resulting mixture was allowed to react at 80°C for 7 hours. After cooling, clean water was added to the reaction solution. Solids obtained by filtration were washed with methanol. These solids were purified with silica gel chromatography (hexane/dichloromethane (75/25)), and dried under a reduced pressure, whereby 1.9 g of white solids were obtained.
As a result of a FD-MS analysis, the resulting white solids were identified as intermediate c.

[Synthesis of compound D-1]

Compound D-1 was synthesized by conducting a reaction in the same manner as in the synthesis of intermediate a, except that 2-bromonaphthalene was used instead of 1,6-dibromopyrene and intermediate c was used instead of 9,9-dimethylfluorene-2-ylboronic acid.
Compound D-1 was identified by a FD-MS analysis. As for Compound D-1, FDMS, a UV absorption maximum wavelength in the toluene solution λ max and a fluorescent emission maximum wavelength were shown below.
FDMS, calcd for C56H40=712, found m/z=712(M+)
UV(PhMe); λmax, 383 nm, FL(PhMe, λex=350nm); λmax, 436nm

Example 2

Compound D-2 was synthesized according to the following scheme:

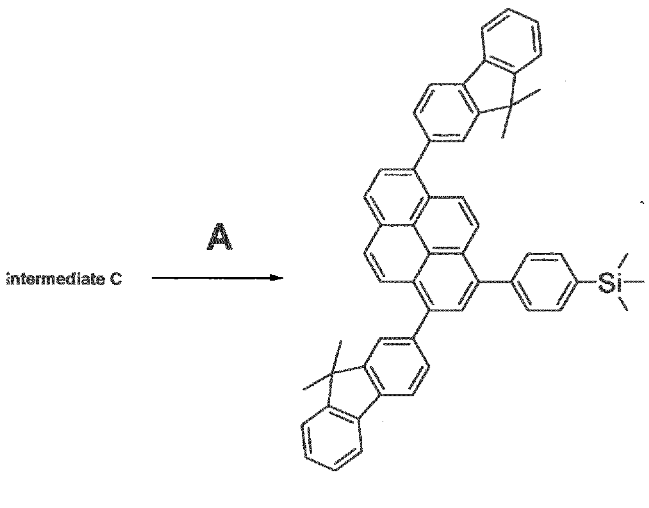


Compound D-2

[Synthesis of compound D-2]

Compound D-2 was synthesized by conducting a reaction in the same manner as in the synthesis of intermediate a, except that 1-bromo-4-(trimethylsilyl)benzene was used instead of 1,6-dibromopyrene and intermediate c was used

instead of 9,9-dimethylfluorene-2-ylboronic acid.
Compound D-2 was identified by a FD-MS analysis.

Example 3

Compound D-3 was synthesized according to the following scheme:

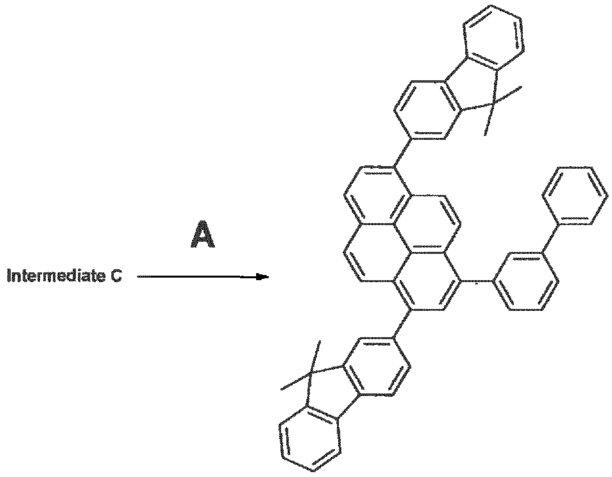


Compound D-3

[Synthesis of compound D-3]

Compound D-3 was synthesized by conducting a reaction in the same manner as in the synthesis of intermediate a, except that 3-bromobiphenyl was used instead of 1,6-dibromopyrene and intermediate c was used instead of 9,9-dimethylfluorene-2-ylboronic acid.
Compound D-3 was identified by a FD-MS analysis.

Example 4

Compound D-4 was synthesized according to the following scheme:

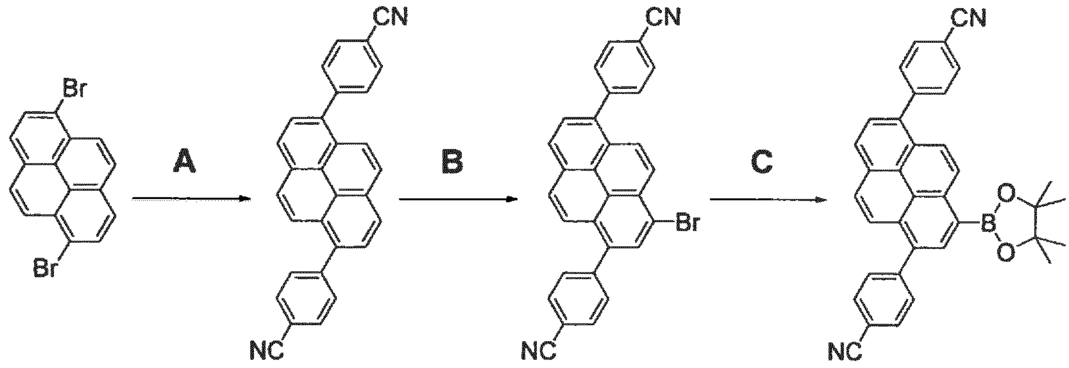


Intermediate d

Intermediate e

Intermediate f

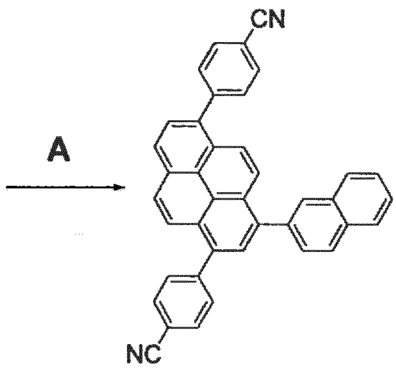


Compound D-4

[Synthesis of intermediate d]

Intermediate d was synthesized by conducting a reaction in the same manner as in the synthesis of intermediate a, except that 4-cyanophenylboronic acid was used instead of 9,9-dimethylfluorene-2-ylboronic acid.
Intermediate d was identified by a FD-MS analysis.

[Synthesis of intermediate e]

Intermediate a was synthesized by conducting a reaction in the same manner as in the synthesis of intermediate b, except that intermediate d was used instead of intermediate a.
Intermediate e was identified by a FD-MS analysis.

[Synthesis of intermediate

Intermediate f was synthesized by conducting a reaction in the same manner as in the synthesis of intermediate c, except that intermediate e was used instead of intermediate b.
Intermediate f was identified by a FD-MS analysis.

[Synthesis of compound D-4]

Compound D-4 was synthesized by conducting a reaction in the same a manner as in the synthesis of intermediate a, except that 2-bromonaphthalene was used instead of 1,6-dibromopyrene and intermediate f was used instead of 9,9-dimethylfluorene-2-ylboronic acid.
Compound D-4 was identified by a FD-MS analysis.

Example 5

Compound D-5 was synthesized according to the following scheme:

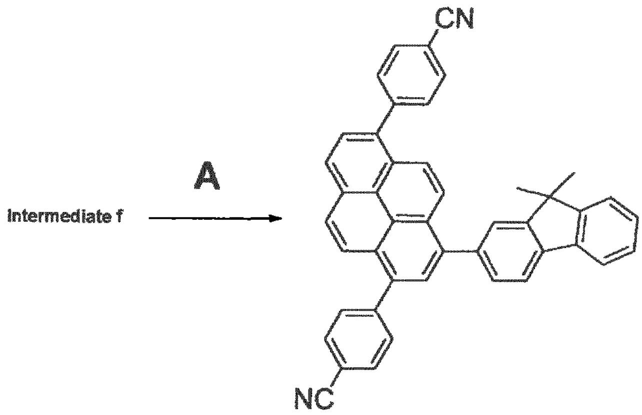


Compound D-5

[Synthesis of compound D-5]

Compound D-5 was synthesized by conducting a reaction in the same manner as in the synthesis of intermediate a, except that 2-bromo-9,9-dimethylfluorene was used instead of 1,6-dibromopyrene and intermediate f was used instead of 9,9-dimethylfluorene-2-ylboronic acid.
Compound D-5 was identified by a FD-MS analysis.

Example 6

Compound D-6 was synthesized according to the following scheme.

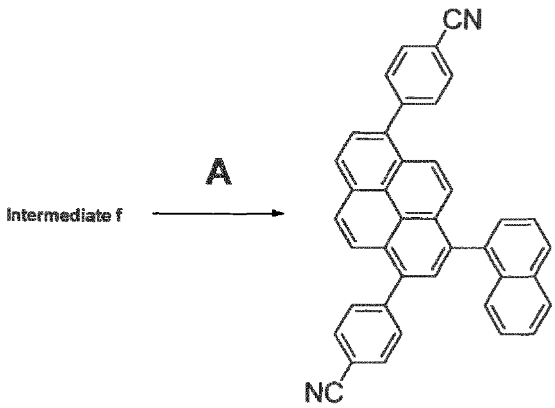


Compound D-6

[Synthesis of compound D-6]

Compound D-6 was synthesized by conducting a reaction in the same manner as in the synthesis of intermediate a, except that 1-bromonaphthalene was used instead of 1,6-dibromopyrene and intermediate f was used instead of 9,9-dimethylfluorene-2-ylboronic acid.
Compound D-6 was identified by a FD-MS analysis.

Example 7

Compound D-7 was synthesized according to the following scheme.

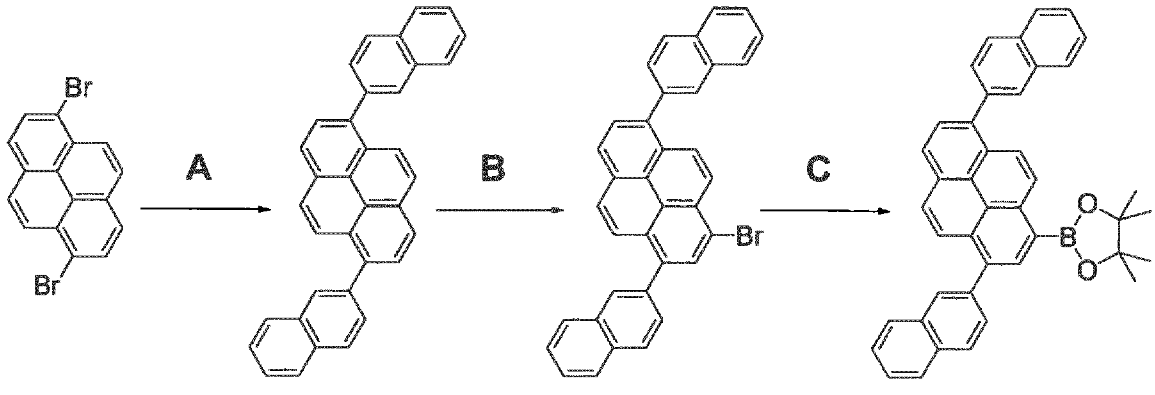


Intermediate g Intermediate h Intermediate i

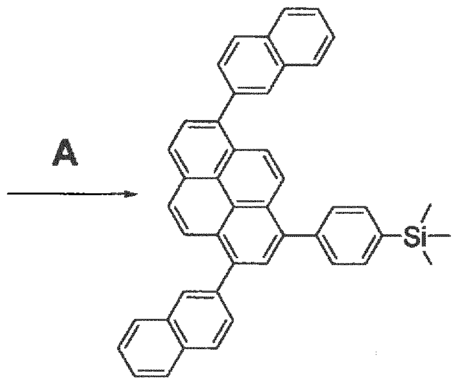


Compound D-7

[Synthesis of intermediate g]

Intermediate g was synthesized by conducting a reaction in the same manner as in the synthesis of intermediate a, except that 2-naphthaleneboronic acid was used instead of 9,9-dimethylfluorene-2-ylboronic acid.
Intermediate g was identified by a FD-MS analysis.

[Synthesis of intermediate h]

Intermediate h was synthesized by conducting a reaction in the same manner as in the synthesis of intermediate b, except that intermediate g was used instead of intermediate a and dimethylformamide was used instead of dichloromethane.
Intermediate h was identified by a FD-MS analysis.

[Synthesis of intermediate i]

Intermediate i was synthesized by conducting a reaction in the same manner as in the synthesis of intermediate c, except that intermediate h was used instead of intermediate b.
Intermediate i was identified by a FD-MS analysis.

[Synthesis of compound D-7]

Compound D-7 was synthesized by conducting a reaction in the same manner as in the synthesis of intermediate a, except that 1-bromo-4-(trimethylsilyl)benzene was used instead of 1,6-dibromopyrene and intermediate i was used instead of 9,9-dimethylfluorene-2-ylboronic acid.
Compound D-7 was identified by a FD-MS analysis.

Example 8

Compound D-8 was synthesized according to the following scheme.

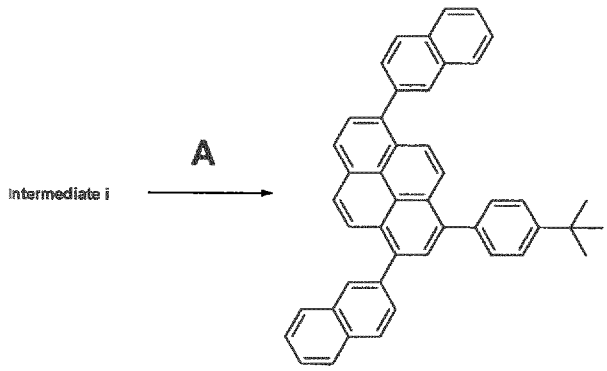


Compound D-8

[Synthesis of compound D-8]

Compound D-8 was synthesized by conducting a reaction the same manner as in the synthesis of intermediate a, except that 1-bromo-4-tert-butylbenzene was used instead of 1,6-dibromopyrene and intermediate i was used instead of 9,9-dimethylfluorene-2-ylboronic acid.
Compound D-8 was identified by a FD-MS analysis.

Example 9

Compound D-9 was synthesized according to the following scheme.

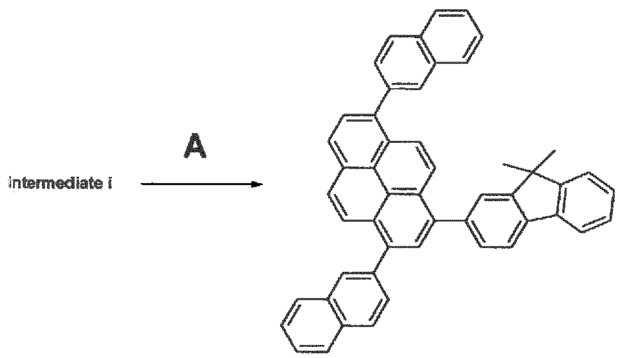


Compound D-9

[Synthesis of compound D-9]

Compound D-9 was synthesized by conducting a reaction in the same manner as in the synthesis of intermediate a, except that 2-bromo-9,9-dimethylfluorene was used instead of 1,6-dibromopyrene and intermediate i was used instead of 9,9-dimethylfluorene-2-ylboronic acid.
Compound D-9 was identified by a FD-MS analysis.

Example 10

Compound D-10 was synthesized according to the following scheme.

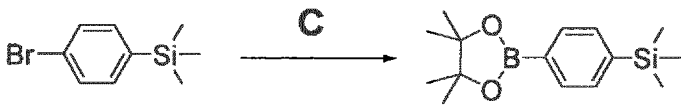


Intermediate j

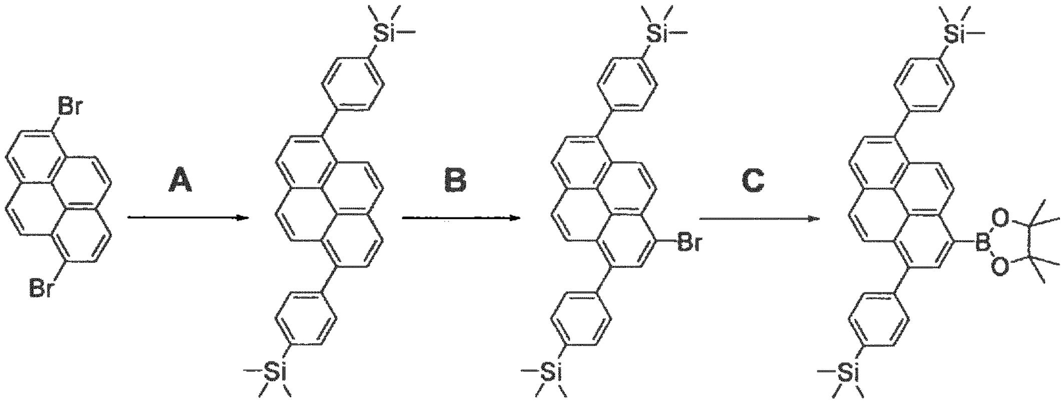


Intermediate k

Intermediate l

Intermediate m

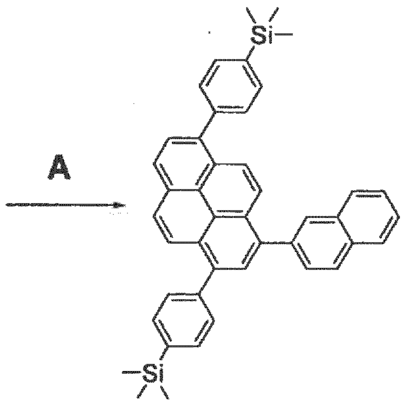


Compound D-10

[Synthesis of intermediate j]

Intermediate j was synthesized by conducting a reaction in the same manner as in the synthesis of intermediate c, except that 1-bromo-4-(trimethylsilyl)benzene was used instead of intermediate b.
Intermediate j was identified by a FD-MS analysis.

[Synthesis of intermediate k]

Intermediate k was synthesized by conducting a reaction in the same manner as in the synthesis of intermediate a, except that intermediate j was used instead of 9,9-dimethylfluorene-2-ylboronic acid.
Intermediate k was identified by a FD-MS analysis.

[Synthesis of intermediate l]

Intermediate l was synthesized by conducting a reaction in the same manner as in the synthesis of intermediate b, except that intermediate k was used instead of intermediate a.
Intermediate l was identified by a FD-MS analysis.

[Synthesis of intermediate m]

Intermediate m was synthesized by conducting a reaction in the same manner as in the synthesis of intermediate c, except that intermediate l was used instead of intermediate b.
Intermediate m was identified by a FD-MS analysis.

[Synthesis of compound D-10]

Compound D-10 was synthesized by conducting a reaction in the same manner as in the synthesis of intermediate a, except that 2-bromonaphthalene was used instead of 1,6-dibromopyrene and intermediate m was used instead of 9,9-dimethylfluorene-2-ylboronic acid.
Compound D-10 was identified by a FD-MS analysis.

Example 11

Compound D-11 was synthesized according to the following scheme.

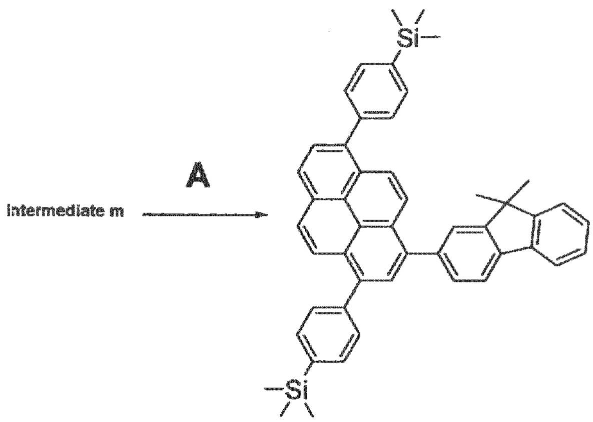


Compound D-11

[Synthesis of compound D-11]

Compound D-11 was synthesized by conducting a reaction in the same manner as in the synthesis of intermediate a, except that 2-bromo-9,9-dimethylfluorene was used instead of 1,6-dibromopyrene and intermediate m was used instead of 9,9-dimethylfluorene-2-ylboronic acid.
Compound D-11 was identified by a FD-MS analysis.

Example 12

Compound D-12 was synthesized according to the following scheme.

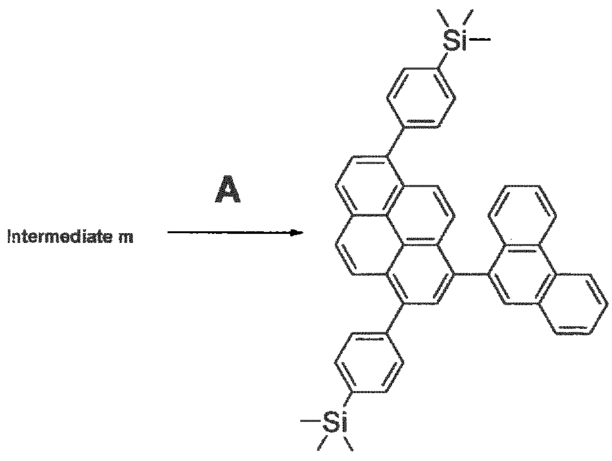


Compound D-12

[Synthesis of compound D-12]

Compound D-12 was synthesized by conducting a reaction in the same manner as in the synthesis of intermediate a, except that 9-bromophenanthrene was used instead of 1,6-dibromopyrene and intermediate m was used instead of 9,9-dimethylfluorene-2-ylboronic acid.
Compound D-12 was identified by a FD-MS analysis.

Example 13

Compound D-13 was synthesized according to the following scheme.

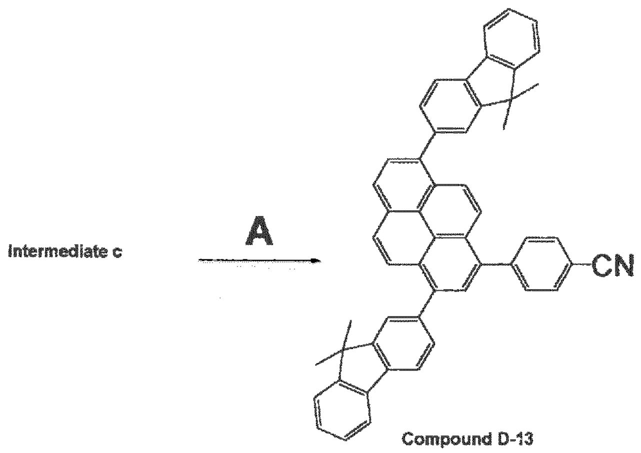


Compound D-13

[Synthesis of compound D-13]

Compound D-13 was synthesized by conducting a reaction in the same manner as in the synthesis of intermediate a, except that 4-bromobenzonitrile was used instead of 1,6-dibromopyrene and intermediate c was used instead of 9,9-dimethylfluorene-2-ylboronic acid.
Compound D-13 was identified by a FD-MS analysis.

Example 14

[Fabrication of organic EL device]

On a glass substrate of 25 mm by 75 mm by 1.1 by 1.1 mm thick, a 120 nm-thick transparent electrode formed of indium tin oxide was provided. This transparent electrode functioned as an anode. Subsequently, this glass substrate was cleaned by irradiating UV rays and ozone. The cleaned glass substrate was installed in a vacuum deposition

apparatus. First, as the hole-injecting layer, compound HT-1 was deposited in a thickness of 50 nm. Subsequently, on the thus formed film, N,N,N'N'-tetrakis(4-biphenyl)-4,4'-benzidine was deposited in a thickness of 45 nm as the hole-transporting layer. Then, 9,10-di(2-naphthyl)anthracene as a host material and compound D-1 as a doping material were co-deposited in a mass ratio of 19:1, whereby an emitting layer with a thickness of 20 nm was formed. On the thus formed emitting layer, compound ET-1 was deposited in a thickness of 30 nm as the electron-injecting layer. Subsequently, lithium fluoride was deposited in a thickness of 1 nm, followed by deposition of aluminum in a thickness of 150 nm, whereby an organic EL device was fabricated. The aluminum/lithium fluoride film functioned as a cathode.

The compound HT-1 and the compound ET-1 are shown below.

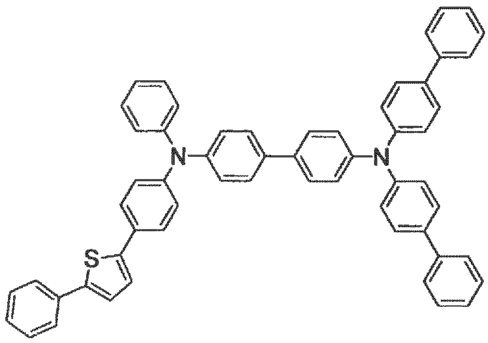

HT-1

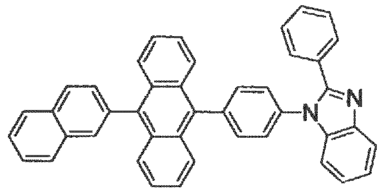

ET-1

For the thus fabricated organic EL device, the chromaticity, the external quantum yield at the time driving at a current density of 10 mA/cm$^2$ and a half life at an initial luminance of 1000 cd/m$^2$ were measured. The results are shown in Table 1.

The 1931 CIE (x,y) chromaticity coordinates: measured by a spectroradiometer (CS1000, produced by MINOLTA).

External quantum yield: Current having a current density of 10 mA/cm$^2$ was applied to the thus obtained organic EL device. Emission spectra thereof were measured with a spectroradiometer(CS-1000, produced by MINOLTA), and external quantum efficiency was calculated by the following equation (1):

$$E.Q.E. = \frac{N_P}{N_E} \times 100$$

$$= \frac{\dfrac{(\pi/10^9)\int \phi(\lambda)\cdot d\lambda}{hc}}{\dfrac{J/10}{e}} \times 100$$

$$= \frac{\dfrac{(\pi/10^9)\Sigma(\phi(\lambda)\cdot(\lambda))}{hc}}{\dfrac{J/10}{e}} \times 100\ (\%)$$

Equation (1)

$N_p$: Number of photons
$N_E$: Number of electrons
$\Pi$: Circular constant = 3.1416
$\lambda$: Wavelength (nm)
$\varphi$: Luminescence intensity (W/sr·m$^2$·nm)
h: Plank constant = 6.63 x $10^{-34}$ (J·s)
c: Light velocity = 3 x $10^8$ (m/s)
J: Current density (mA/cm$^2$)
e: Charge = 1.6 x $10^{-19}$ (C)

Examples 15 to 17

Organic EL devices were fabricated and evaluated in the same manner as in Example 14, except that the doping materials were changed as shown in Table 1. The results are shown in Stable 1.

Comparative Examples 1

An organic EL device was fabricated in the same manner as in Example 13, except that H-1 was used instead of D-1. The thus obtained organic EL device was evaluated in the same manner as in Example 14, and the results obtained are shown in Table 1.

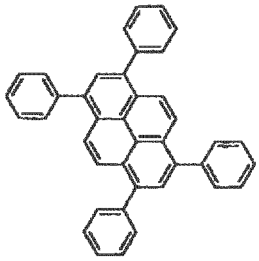

H−1

Table 1

| | Doping material | Chromaticity | External quantum yield (%) | Half life (hr) |
|---|---|---|---|---|
| Example 14 | D-1 | (0.147,0.071) | 6.3 | 1700 |
| example 15 | D-3 | (0.149,0.065) | 6.3 | 1400 |
| Example 16 | D-9 | (0.147,0.061) | 6.3 | 1100 |
| Example 17 | D-13 | (0.147,0.083) | 6.8 | 2200 |
| Com. Ex. 1 | H-1 | (0.153,0.050) | 3.8 | 150 |

From Table 1, it can be understood that the devices of the examples could maintain a high luminous efficiency and had a long life while exhibiting a high degree of color purity. By using the devices of the examples, a display device having improved color reproducibility with low power consumption can be realized.

INDUSTRIAL APPLICABILITY

The organic EL device of the invention can be applied to products which require a high luminous efficiency at a low voltage. As the application examples, a display apparatus, a display, illuminating equipment, a printer light source, backlight of a liquid crystal display device or the like can be given. It can also be applied in the fields of a traffic sign, a signboard, interior decorating goods or the like. As the display apparatus, an energy-saving or highly-visible flat panel display can be mentioned. As for the printer light source, it can be used as the light source for a laser beam printer. By using the device of the invention, the volume of an apparatus can be significantly reduced. As for the illuminating equipment or the backlight, energy-saving effects can be expected by using the organic EL device of the invention.

Although only some exemplary embodiments and/or examples of this invention have been described in detail above, those skilled in the art will readily appreciate that many modifications are possible in the exemplary embodiments and/or examples without materially departing from the novel teachings and advantages of this invention. Accordingly, all such modifications are intended to be included within the scope of this invention.

The documents described in the specification are incorporated herein by reference in its entirety.

## Claims

1. A pyrene derivative represented by the following formula (1):

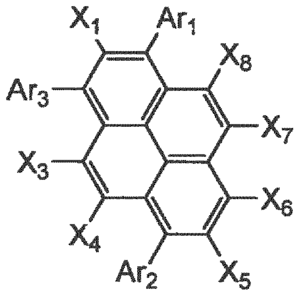

(1)

wherein $Ar_1$, $Ar_2$ and $Ar_3$ are independently a substituted or unsubstituted aryl group; and
$X_1$, and $X_3$ to $X_8$ are independently a hydrogen atom, a substituted or unsubstituted aryl group, a substituted or unsubstituted alkyl group or a substituted or unsubstituted cycloalkyl group;
excluding a derivative in which $X_6$ is a substituted or an unsubstituted aryl group and all of $X_1$ and $X_3$ to $X_5$, $X_7$ and $X_8$ are hydrogen atoms and a derivative in which the substituted or unsubstituted aryl groups of $Ar_1$, $Ar_2$ and $Ar_3$ are the same.

2. The pyrene derivative according to claim 1, wherein $Ar_1$ and $Ar_2$ are the same and $Ar_3$ is different from $Ar_1$ and $Ar_2$.

3. The pyrene derivative according to claim 1, wherein $Ar_1$, $Ar_2$ and $Ar_3$ are different from each other.

4. The pyrene derivative according to claim 1, wherein $X_6$ is a hydrogen atom.

5. The pyrene derivative according to claim 4, wherein $Ar_1$ and $Ar_2$ are the same. .

6. The pyrene derivative according to any of claims 1 to 5, wherein $Ar_1$ and are independently a substituent represented by the following formula (2):

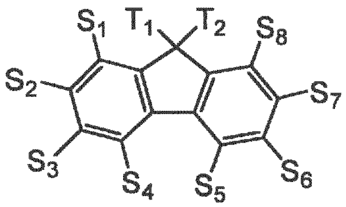

(2)

wherein $S_1$ to to $S_8$ are independently a hydrogen atom, a substituted or unsubstituted aryl group, a substituted or unsubstituted alkyl group, a substituted or unsubstituted cycloalkyl group or a substituted or unsubstituted alkenyl group, or at least two adjacent groups of $S_1$ to $S_8$ are combined to form a saturated or unsaturated ring structure that may have a substituent;
$T_1$ and $T_2$ are independently a hydrogen atom, a substituted or unsubstituted aryl group, a substituted or unsubstituted alkyl group, a substituted or unsubstituted cycloalkyl group or a substituted or unsubstituted silyl group; and
the substituent represented by the formula (2) is bonded to the pyrene skeleton of the pyrene derivative through one of $S_1$ to $S_8$ as a single bond or is bonded to the pyrene skeleton of the pyrene derivative by a single bond in any one of bonding positions of a saturated or unsaturated ring structure which is formed by adjacent groups of $S_1$ to $S_8$.

7. The pyrene derivative according to claim 6, wherein $S_2$ is bonded to the pyrene skeleton of the pyrene derivative represented by the formula (1) as a single bond.

8. The pyrene derivative according to any of claims 1 to 5, wherein $Ar_1$ and $Ar_2$ are independently a naphthyl group, an aryl group having a naphthyl group, an aryl group having a substituted or unsubstituted silyl group or an aryl group having a cyano group.

**9.** An organic electroluminescence device comprising a pair of electrodes and one or more organic compound layers comprising an emitting layer therebetween,
wherein the emitting layer comprises the pyrene derivative according to any of claims 1 to 8 in an amount of 0.1 to 50 mass%.

**10.** The organic electroluminescence device according to claim 9, wherein the emitting layer comprises the pyrene derivative in an amount of 0.1 to 20 mass%.

## INTERNATIONAL SEARCH REPORT

International application No.
PCT/JP2010/007361

A. CLASSIFICATION OF SUBJECT MATTER
*C07C13/66*(2006.01)i, *C07C255/50*(2006.01)i, *C07C255/51*(2006.01)i, *C07F7/08*(2006.01)i, *C09K11/06*(2006.01)i, *H01L51/50*(2006.01)i

According to International Patent Classification (IPC) or to both national classification and IPC

B. FIELDS SEARCHED

Minimum documentation searched (classification system followed by classification symbols)
C07C13/00, C07C255/00, C07F7/00, C09K11/00, H01L51/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)
CA/REGISTRY(STN)

C. DOCUMENTS CONSIDERED TO BE RELEVANT

| Category* | Citation of document, with indication, where appropriate, of the relevant passages | Relevant to claim No. |
|---|---|---|
| P,X | WO 2010/134352 A1 (Idemitsu Kosan Co., Ltd.), 25 November 2010 (25.11.2010), pages 53 to 54 (Family: none) | 1-10 |
| X | JP 2009-290051 A (Toray Industries, Inc.), 10 December 2009 (10.12.2009), pages 11 to 14 (Family: none) | 1-10 |
| X | JP 2009-246354 A (Toray Industries, Inc.), 22 October 2009 (22.10.2009), pages 15, 18 to 20 (Family: none) | 1-10 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>20 January, 2011 (20.01.11) | Date of mailing of the international search report<br>01 February, 2011 (01.02.11) |
| Name and mailing address of the ISA/<br>Japanese Patent Office<br>Facsimile No. | Authorized officer<br><br>Telephone No. |

Form PCT/ISA/210 (second sheet) (July 2009)

INTERNATIONAL SEARCH REPORT

International application No.
PCT/JP2010/007361

C (Continuation). DOCUMENTS CONSIDERED TO BE RELEVANT

| Category* | Citation of document, with indication, where appropriate, of the relevant passages | Relevant to claim No. |
|---|---|---|
| X | WO 2009/116456 A1 (Toray Industries, Inc.), 24 September 2009 (24.09.2009), page 27 (Family: none) | 1-10 |
| X | WO 2008/108260 A1 (Toray Industries, Inc.), 12 September 2008 (12.09.2008), pages 11 to 18 & JP 2008-252063 A | 1-10 |
| X | WO 2008/108256 A1 (Toray Industries, Inc.), 12 September 2008 (12.09.2008), pages 7 to 15 & US 2010/0038634 A1 & EP 2128217 A1 | 1-10 |
| X | JP 2008-195841 A (Toray Industries, Inc.), 28 August 2008 (28.08.2008), page 28 (Family: none) | 1-10 |
| X | JP 2008-159843 A (Toray Industries, Inc.), 10 July 2008 (10.07.2008), pages 20, 23 (Family: none) | 1-10 |
| X | WO 2007/145136 A1 (Toray Industries, Inc.), 21 December 2007 (21.12.2007), pages 16 to 19 & US 2010/0163852 A1 & EP 2028249 A1 | 1-10 |
| X | JP 2007-208165 A (Toray Industries, Inc.), 16 August 2007 (16.08.2007), pages 15 to 16, 19 to 21, 23 to 34, 36 to 38 (Family: none) | 1-10 |
| X | JP 2007-191603 A (Toray Industries, Inc.), 02 August 2007 (02.08.2007), page 23 (Family: none) | 1-10 |
| X | JP 2007-131723 A (Toray Industries, Inc.), 31 May 2007 (31.05.2007), pages 7 to 11 (Family: none) | 1-10 |
| X | JP 2007-131722 A (Toray Industries, Inc.), 31 May 2007 (31.05.2007), page 20 (Family: none) | 1-10 |
| X | WO 2007/029798 A1 (Toray Industries, Inc.), 15 March 2007 (15.03.2007), pages 9 to 11, 42 to 47, 49 to 55 & US 2009/0096356 A1 & EP 1942171 A1 | 1-10 |

Form PCT/ISA/210 (continuation of second sheet) (July 2009)

INTERNATIONAL SEARCH REPORT

International application No.
PCT/JP2010/007361

C (Continuation). DOCUMENTS CONSIDERED TO BE RELEVANT

| Category* | Citation of document, with indication, where appropriate, of the relevant passages | Relevant to claim No. |
|---|---|---|
| X | WO 2006/090772 A1 (Toray Industries, Inc.), 31 August 2006 (31.08.2006), pages 10 to 15, 17 to 18, 20 to 22, 24 to 29 & JP 2006-265515 A & US 2009/0066245 A1 & EP 1852486 A1 | 1-10 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2003234190 A
- JP 2005126431 A
- JP 2009004351 A


### Non-patent literature cited in the description


- *Chem. Rev.,* 1995, vol. 95 (7), 2457
- Experimental Chemistry. vol. 24, 61-90
- *J. Org. Chem.,* 1995, vol. 60, 7508